# EUROPEAN PATENT APPLICATION

(11) **EP 4 129 314 A1**
(43) Date of publication of application: **08.02.2023**
(21) Application number: 21791867.1
(22) Date of filing: 23.04.2021
(51) Int. Cl.: A61K 36/288, A01P 7/02, A61P 27/02, A61P 37/08, A61P 17/00

(54) **USE OF DANDELION AND MONOMER COMPOUNDS THEREOF FOR KILLING MITES**

(30) Priority: 24.04.2020 CN 202010334090
(71) Applicant: Smilebiotek Zhuhai Limited, Zhuhai, Guangdong 519000 (CN)
(72) Inventor: ZHANG, Yan, Zhuhai, Guangdong 519000 (CN)
(74) Representative: HGF
(86) International application number: PCT/CN2021/089110
(87) International publication number: WO 2021/213485

(57) **Abstract**

Use of dandelion and monomer compounds thereof for preparing products for killing mites. Dandelion can reduce the survival time of mites. The dandelion monomers, especially taraxerol, diosmetin and taraxasterol acetate, can significantly shorten the survival time of demodex, and can be used for preparing products for killing and inhibiting mites.

## Description

### TECHNICAL FIELD

The present invention relates to the technical field of pharmaceuticals and daily care, and in particular to use of Herba Taraxaci and monomer compounds thereof, specifically taraxerol, diosmetin and taraxasterol acetate, in killing mites.

### BACKGROUND

Mites are a class of tiny animals belonging to Arthropoda, Arachnida, Latigastra, generally having a body size of about 0.5 mm, some as small as 0.1 mm, and most species have a body size of less than 1 mm. It is found that mites are closely related to the health status of human beings and animals (such as dogs, cats and other companion animals). For example, mites such as gamasid mites (*Mesostigmata* spp.), chiggers, itch mite (*Sarcoptes scabiei*)*, Demodex* mites (*Demodex* spp.), flour mites (*Acarus siro*)*,* dust mite and *Pyemotes spp.* are hematophagic and may harm the skin, causing "acne rosacea" or demodicosis, hypersensitivity, urinary acariasis, pulmonary acariasis, intestinal acariasis, scabies and the like, which seriously endanger the health of human beings and animals. *Demodex* mites (belonging to *Arachnid, Acarina*) are microscopic ectoparasites that usually affect the pilosebaceous unit of the skin. In the wide range of reported species, *D. folliculorum* and *D. brevis* have been found on the body surface of humans, and mainly live in the hair follicles, sebaceous glands and meibomian glands. *Demodex* mites feed on epithelial cells of hair follicle, causing follicle dilatation and hair loss, manifesting clinically as blepharitis marginalis, meibomian gland dysfunction, madarosis, abnormal eyelash alignment, conjunctivitis and blepharoconjunctivitis, pterygium, keratitis, basal cell carcinoma of eyelid and the like. *Demodex* mites may also feed on lipids, causing xerophthalmia. In addition, *Demodex* mites may also cause mechanical obstruction of meibomian gland ducts, causing difficulty in lipid excretion and excessive retention of secretions, leading to the formation of chalazion. The clinical manifestations of the ocular diseases caused by *Demodex* infestation mainly include: recurrent red and itch eyes, dry eyes, burning eyes, foreign body sensation, photophobia and increased secretion; the diseases may also be accompanied by recurrent eyelash loss; blurred vision and decreased vision may occur in severe cases affecting the cornea. In addition to the above ocular diseases, a number of studies in recent years have reported that *Demodex* infestation is associated with a variety of common skin diseases, including seborrhoeic dermatitis, acne, acne rosacea, pityriasis folliculorum, perioral dermatitis, demodicosis, basal cell carcinoma and the like.

In particular, reports where the association of mites with ocular diseases has been confirmed include:
Human Permanent Ectoparasites; Recent Advances on Biology and Clinical Significance of Demodex Mites: Narrative Review Article (Iranian journal of parasitology 12(1):12-21) has shown that *D. brevis* lives in the sebaceous glands and meibomian glands and *D. folliculorum* often occupies the hair follicle area of human eyelashes, of which both will cause ocular diseases after infecting the facial skin.

Ocular Demodicosis as a Potential Cause of Ocular Surface Inflammation (*Cornea 36 Suppl 1*(Suppl 1):s9-s14) demonstrates that *D. brevis* and *D. folliculorum* are two *Demodex* species that cause ocular demodicosis in humans, and there is a positive correlation between human age and the risk of the disease and a strong positive correlation between ocular demodicosis and ocular surface inflammatory conditions.

*Demodex* species in human ocular disease: new clinicopathological aspects (International ophthalmology 37(1):303-312) has shown that *D. brevis* and *D. folliculorum* are associated with the pathogenesis of external ocular diseases, and ocular demodicosis can cause an imbalance in the extraocular environment.

Ocular Demodex: a systematic review of the clinical literature (Ophthalmic & physiological optics: the journal of the British College of Ophthalmic Opticians (Optometrists) 40(4):389-432) has shown that *Demodex* infestation is a potential cause of several ocular surface diseases, and the parasitism of *Demodex* mites on the anterior structures of the eyes such as eyelids, eyelashes and the ocular surface can lead to the ocular demodicosis in humans, and the incidence is positively correlated with age.

The relationship between demodex and ocular discomfort (Investigative ophthalmology & visual science 51(6):2906-2911) suggested that the number of *Demodex* mites is positively correlated with the severity of ocular diseases and the age of patients.

*Demodex* mites (Clinics in dermatology 32(6):739-743) suggested that *Demodex* infestation can cause chronic blepharitis marginalis, and the prevalence of chronic blepharitis marginalis is positively correlated with the number of mites and the age of patients.

Quantitative Analysis of the Bacteria in Blepharitis With *Demodex* Infestation (*Frontiers in microbiology* 9:1719) has shown that *D. brevis* and *D. folliculorum* infestations can lead to the occurrence of ocular diseases, the prevalence is positively correlated with the number of mites and the age of patients, and the *Demodex* mites are carriers of *Bacillus spp.,* which can play a role as co-pathogens in the pathogenesis of blepharitis marginalis.

*Bacillus oleronius* and *Demodex* mite infestation in patients with chronic blepharitis (Clinical microbiology and infection: the official publication of the European Society of Clinical Microbiology and Infectious Diseases 18(10):1020-1025) suggested that *Demodex* mites may cause the occurrence of blepharitis marginalis and are carriers of *Bacillus spp.,* which can play a role as co-pathogens in the development of blepharitis marginalis.

Correlation between ocular *Demodex* infestation and serum immunoreactivity to Bacillus proteins in patients with Facial rosacea (Ophthalmology 117(5):870-877.e871) suggested that the incidence of ocular *Demodex* infestation in patients with xerophthalmia is lower than that in patients without xerophthalmia, and that the mite infestation and bacterial infection can coexist in ocular surface inflammation.

High prevalence of demodex brevis infestation in chalazia (American journal of ophthalmology 157(2):342-348.e341) has shown that the incidence of demodicosis caused by *D. brevis* is high in adults and pediatric patients with chalazion, and the ocular demodicosis is a major cause of chalazion.

Diseases caused by mites (such as ocular diseases and skin diseases) have not yet received sufficient clinical attention, and are often misdiagnosed as bacterial diseases and the like, while the conventional antibacterial treatments usually have little curative effect.

### SUMMARY

In a first aspect of the present invention, provided is use of Herba Taraxaci in preparing a product for killing mites.

Specifically, the Herba Taraxaci can be used as the sole acaricidal active ingredient, or can also be used in combination with other ingredients with the same or different activities for killing mites.

In a second aspect of the present invention, provided is use of a Herba Taraxaci extract in preparing a product for killing mites.

Specifically, the Herba Taraxaci extract can be obtained by suitable extraction methods for traditional Chinese medicine known in the art, such as one or more of decoction, maceration, percolation, reflux, steam distillation, ultrasonic extraction and the like. In an embodiment of the present invention, the Herba Taraxaci extract can be obtained by decoction.

Specifically, the Herba Taraxaci extract is prepared by a method comprising:
(1) soaking Herba Taraxaci in an extraction solvent; and
(2) heating the mixture of step (1) to boiling, then decocting and filtering.

In an embodiment of the present invention, the extraction solvent is water.

Specifically, the time for soaking is 1-24 hours (e.g., 1, 2, 3, 4, 5, 6, 12, 18 or 24 hours).

Specifically, the time for decocting is 10-60 min (e.g., 10, 15, 20, 25, 30, 35, 40, 45, 50, 55 or 60 min).

Specifically, the method may further comprise:
(3) heating the filter residue obtained in step (2) and an extraction solvent to boiling, then decocting and filtering; and combining the filtrates obtained in steps (2) and (3).

Optionally, the method may further comprise one or more of the processing procedures of mixing, concentrating, quantifying or sterilizing the obtained filtrate and the like.

Specifically, the Herba Taraxaci extract comprises one or more of taraxasterol, taraxerol, taraxerone, diosmetin, taraxasterol acetate, caffeic acid, taraxeryl acetate and lupenone.

More specifically, the Herba Taraxaci extract comprises one or more of taraxerol, diosmetin and taraxasterol acetate.

In a third aspect of the present invention, provided is use of a monomer compound of Herba Taraxaci, or a salt, an isomer, a solvate or a derivative thereof in preparing a product for killing mites.

Specifically, the salt, the isomer, the solvate and the derivative of the monomer compound of Herba Taraxaci are pharmaceutically acceptable salt, isomer, solvate and derivative.

Specifically, the monomer compound is selected from one or more of taraxasterol, taraxerol, taraxerone, diosmetin, taraxasterol acetate, caffeic acid, taraxeryl acetate and lupenone.

More specifically, the monomer compound is selected from one or more of taraxerol, diosmetin and taraxasterol acetate.

In a fourth aspect of the present invention, provided is use of taraxerol in preparing a product for killing mites.

In a fifth aspect of the present invention, provided is use of diosmetin in preparing a product for killing mites.

In a sixth aspect of the present invention, provided is use of taraxasterol acetate in preparing a product for killing mites.

Specifically, the product for killing mites described above can be used for therapeutic and/or prophylactic purposes, and also for non-therapeutic and/or non-prophylactic purposes.

Specifically, the mites described above can be one or more of *Demodex spp., Dermatophagoides pteronyssinus, Sarcoptes scabiei.* and the like. In an embodiment of the present invention, the mites described above are *Demodex spp.,* such as *D. folliculorum* and *D. brevis.*

In an embodiment of the present invention, the product for killing mites described above is a pharmaceutical composition.

Specifically, the pharmaceutical composition described above further comprises a pharmaceutically acceptable excipient(s).

Specifically, the pharmaceutical composition described above is used for preventing and/or treating a disease caused by mite infestation.

Specifically, the disease described above may be an ocular disease, a skin disease, an allergic disease and the like.

Specifically, the ocular disease described above may be one or more of blepharitis marginalis, meibomian gland dysfunction, tarsitis, madarosis, abnormal eyelash alignment, glaucoma, cataract, ocular folliculitis, conjunctivitis, blepharoconjunctivitis, pterygium, keratitis, eyelid laxity and ectropion, basal cell carcinoma of eyelid, xerophthalmia, chalazion and the like; the ocular disease may have one or more symptoms selected from: red and itchy eyes, dry eyes, burning eyes, foreign body sensation, photophobia, increased eye discharge, loss of eyelashes, blurred vision, decreased vision and the like.

Specifically, the skin disease described above may be one or more of seborrhoeic dermatitis, acne, acne rosacea, pityriasis folliculorum, perioral dermatitis, demodicosis, gaile, basal cell carcinoma and the like.

Specifically, the allergic disease described above may be one or more of allergic asthma, allergic rhinitis, allergic dermatitis and the like.

Specifically, the pharmaceutical composition described above may be in any dosage form suitable for administration, such as a topical preparation, in particular, an ophthalmic preparation, a skin topical preparation and the like.

Specifically, the ophthalmic preparation described above may be an eye drop, an eye ointment, an ophthalmic gel, an ophthalmic emulsion, an ophthalmic suspension, an ophthalmic film, a collyrium, an intraocular injection and the like.

Specifically, the ophthalmic preparation may comprise a pharmaceutically acceptable excipient(s) such as a pH regulator, a co-solvent, an osmotic pressure regulator, a viscosity regulator, an antioxidant, a bacteriostatic preservative, a buffer, a suspending agent, a local anesthetic, a surfactant, a solubilizer, a wetting agent, an emulsifier, a stabilizer, a filler, a protective agent, a solvent and the like.

Specifically, the skin topical preparation described above may be an aerosol, a powder, a lotion, a tincture, a liniment, a film coating agent, an ointment, a gel, a paste, an emulsion and the like. Specifically, the dosage forms of the pharmaceutical composition described above can be prepared according to conventional production methods in the field of pharmaceuticals. For example, ophthalmic preparation techniques can refer to those found in Ophthalmic Drugs and Preparation Techniques (China Light Industry Press, 2010), Development and Production of Eye Drops (Chemical Industry Press, 2009), etc.

Specifically, the pharmaceutical composition described above may comprise 0.01%-99.5% (e.g., 0.01%, 0.1%, 0.5%, 1%, 2%, 3%, 4%, 5%, 6%, 7%, 8%, 9%, 10%, 20%, 30%, 40%, 50%, 60%, 70%, 80%, 90%, 95%, 99% or 99.5%) by weight of the active ingredient.

Specifically, the pharmaceutical composition described above can be used in humans, and can also be used as a veterinary drug in non-human animals, such as non-human mammals, e.g., companion animals (e.g., dogs, cats, rabbits, mice, etc.), livestock animals (e.g., horses, cows, sheep, pigs, dogs, rabbits, etc.) and the like.

In another embodiment of the present invention, the product for killing mites described above is a cosmetic product.

Specifically, the cosmetic product described above further comprises a cosmetically acceptable excipient(s).

Specifically, the cosmetic product described above may be a cosmetic product used for the face, such as a facial cleanser, a soap, a skin softener, a toner, a skin care lotion, a jelly lotion, a facial cream, a sunscreen cream, an essence, a facial mask, a gel, a foundation and a scrub.

Specifically, the cosmetic product described above may be a cosmetic product used for parts of the body other than the face, such as a neck cream, a shampoo, a shower gel, a hair conditioner, a body lotion, a scrub and the like.

Specifically, the cosmetic product described above may also be a cosmetic product used for the eyes and periocular region, such as an eye cream, a mascara, an eyeliner powder, a cream eyeliner, an eyeliner pencil, an eye shadow powder, an eye shadow cream, an eyebrow pencil, a brow powder and the like.

Specifically, the various forms of the cosmetic product described above can be prepared according to conventional production methods in the field of cosmetics.

Specifically, the cosmetic product described above may comprise 0.01%-99.5% (specifically, 0.01%, 0.1%, 0.5%, 1%, 2%, 3%, 4%, 5%, 6%, 7%, 8%, 9%, 10%, 20%, 30%, 40%, 50%, 60%, 70%, 80%, 90%, 95%, 99% or 99.5%) by weight of the active ingredient.

In another embodiment of the present invention, the product for killing mites described above is an acaricide which can be used for killing and controlling mites that may live in articles in the living environment (e.g., pillow case, pillow inner, bed sheet, bedding, mattress, clothing, carpet, cushion, sofa, summer sleeping mat, plush toy, air conditioner and the like).

Specifically, the acaricide described above may comprise any suitable excipient that can achieve the desired properties.

Specifically, the acaricide described above may be in a form of a spray, a lotion, a paster, a small packet and the like.

Specifically, the various forms of the acaricide described above can be prepared according to conventional production methods in the field of daily care products.

Specifically, the acaricide described above may comprise 0.01%-99.5% (specifically, 0.01%, 0.1%, 0.5%, 1%, 2%, 3%, 4%, 5%, 6%, 7%, 8%, 9%, 10%, 20%, 30%, 40%, 50%, 60%, 70%, 80%, 90%, 95%, 99% or 99.5%) by weight of the active ingredient.

The present invention further provides a method for preventing and/or treating a disease caused by mite infestation, comprising administering to a subject in need thereof Herba Taraxaci, or an extract thereof, a monomer compound thereof (in particular, taraxerol, diosmetin and taraxasterol acetate) or the pharmaceutical composition described above.

Specifically, the disease described above may be an ocular disease, a skin disease, an allergic disease and the like.

Specifically, the ocular disease described above may be one or more of blepharitis marginalis, meibomian gland dysfunction, tarsitis, madarosis, abnormal eyelash alignment, glaucoma, cataract, ocular folliculitis, conjunctivitis, blepharoconjunctivitis, pterygium, keratitis, eyelid laxity and ectropion, basal cell carcinoma of eyelid, xerophthalmia, chalazion and the like; the ocular disease may have one or more symptoms selected from: red and itchy eyes, dry eyes, burning eyes, foreign body sensation, photophobia, increased eye discharge, loss of eyelashes, blurred vision, decreased vision and the like.

Specifically, the skin disease described above may be one or more of seborrhoeic dermatitis, acne, acne rosacea, pityriasis folliculorum, perioral dermatitis, demodicosis, gaile, basal cell carcinoma and the like.

Specifically, the allergic disease described above may be one or more of allergic asthma, allergic rhinitis, allergic dermatitis and the like.

Specifically, the subject described above can be any animal receiving the prophylaxis and/or treatment, in particular, a mammal, such as a human, a cat, a dog, a rabbit, a mouse, a horse, a cow, a sheep, a pig and the like. In one embodiment of the present invention, the subject described above is a human; and in another embodiment of the present invention, the subject described above is a non-human animal.

Specifically, the dose of the Herba Taraxaci, or the extract thereof, the monomer compound thereof (in particular, taraxerol, diosmetin and taraxasterol acetate) or the pharmaceutical composition described above may vary according to the route of administration, the age and body weight of the subject, the disease to be treated in the subject and the severity, etc., and may be administered in one or more doses.

In a seventh aspect of the present invention, provided is use of Herba Taraxaci, a Herba Taraxaci extract, a monomer compound of Herba Taraxaci, or a salt, an isomer, a solvate or a derivative thereof in preparing a medicament for treating and/or preventing an ocular disease caused by mites. In an eighth aspect of the present invention, provided is a method for treating and/or preventing an ocular disease caused by mites, comprising administering to eyes of a subject in need thereof Herba Taraxaci, a Herba Taraxaci extract, a monomer compound of Herba Taraxaci, or a salt, an isomer, a solvate or a derivative thereof.

Specifically, the subject described above can be any animal receiving the prophylaxis and/or treatment, in particular, a mammal, such as a human, a cat, a dog, a rabbit, a mouse, a horse, a cow, a sheep, a pig and the like. In one embodiment of the present invention, the subject described above is a human; and in another embodiment of the present invention, the subject described above is a non-human animal.

Specifically, the ophthalmic administration is an administration in a form of an eye drop, an eye ointment, an ophthalmic gel, an ophthalmic emulsion, an ophthalmic suspension, an ophthalmic film, a collyrium or an intraocular injection.

Specifically, the ocular disease described above can be one or more of blepharitis marginalis, meibomian gland dysfunction, tarsitis, madarosis, abnormal eyelash alignment, glaucoma, cataract, ocular folliculitis, conjunctivitis, blepharoconjunctivitis, pterygium, keratitis, eyelid laxity and ectropion, basal cell carcinoma of eyelid, xerophthalmia, chalazion and the like; the ocular disease may have one or more symptoms selected from: red and itchy eyes, dry eyes, burning eyes, foreign body sensation, photophobia, increased eye discharge, loss of eyelashes, blurred vision, decreased vision and the like. The association of the mites with the ocular diseases has been demonstrated in the following documents:
Global divergence of the human follicle mite Demodex folliculorum: Persistent associations between host ancestry and mite lineages, Proceedings of the National Academy of Sciences of the United States of America, 112(52):15958-15963 (blepharitis marginalis);
Human Permanent Ectoparasites; Recent Advances on Biology and Clinical Significance of Demodex Mites: Narrative Review Article, Iranian journal of parasitology, 12(1):12-21 (tarsitis and blepharitis marginalis);
Ocular Demodicosis as a Potential Cause of Ocular Surface Inflammation, *Cornea 36 Suppl* 7(Suppl 1):S9-S14 (blepharitis marginalis, keratitis, conjunctivitis, blepharoconjunctivitis, tarsitis, meibomian gland dysfunction and basal cell carcinoma of eyelid);
Demodex species in human ocular disease: new clinicopathological aspects, International ophthalmology, 37(1):303-312 (external ophthalmopathy, xerophthalmia, blepharitis, trichiasis, eyelid laxity and ectropion, and blepharitis marginalis);
Ocular Demodex: a systematic review of the clinical literature, Ophthalmic & physiological optics: the journal of the British College of Ophthalmic Opticians (Optometrists), 40(4):389-432 (meibomian gland dysfunction, eyelid conjunctivitis, blepharoconjunctivitis, herpes simplex keratitis, recurrent keratitis, herpes keratitis, xerophthalmia, blepharitis, tarsitis, chalazion, basal cell carcinoma, glaucoma, cataract, ocular folliculitis, chronic and recurrent inflammation of eyelid margin, limbitis, inflammation of the eyelids, misdirection of eyelashes and laxity);
The relationship between Demodex and ocular discomfort, Investigative ophthalmology & visual science, 51(6):2906-2911 (meibomian gland dysfunction, blepharitis marginalis (chronic blepharitis marginalis), glaucoma);
Demodex mites, Clinics in dermatology, 32(6):739-743 (blepharitis marginalis, short blepharitis marginalis, chronic blepharitis marginalis, follicular blepharitis marginalis, anterior blepharitis, posterior blepharitis, meibomian gland dysfunction, and keratoconjunctivitis);
Quantitative Analysis of the Bacteria in Blepharitis With *Demodex* Infestation, *Frontiers in microbiology,* 9:1719 (blepharitis marginalis, chronic blepharitis marginalis, mixed blepharitis marginalis, blepharitis, anterior blepharitis, posterior blepharitis, meibomian gland dysfunction); Bacillus oleronius and Demodex mite infestation in patients with chronic blepharitis, Clinical microbiology and infection: the official publication of the European Society of Clinical Microbiology and Infectious Diseases, 18(10):1020-1025 (chronic blepharitis marginalis, moderate blepharitis marginalis, severe blepharitis marginalis, blepharitis marginalis, conjunctivitis, meibomian gland dysfunction, and blepharitis);
Correlation between ocular Demodex infestation and serum immunoreactivity to Bacillus proteins in patients with Facial rosacea, Ophthalmology, 117(5):870-877.e871 (blepharitis marginalis, xerophthalmia, ocular surface inflammation, and conjunctivochalasis);
High prevalence of Demodex brevis infestation in chalazia, American journal of ophthalmology, 157(2):342-348.e341 (chalazion, blepharitis marginalis, conjunctivitis, and keratitis);
Dissecting lipid metabolism in meibomian glands of humans and mice: an integrative study reveals a network of metabolic reactions not duplicated in other tissues, Biochimica et Biophysica Acta (BBA)-Molecular and Cell Biology of Lipids, 1861(6):538-553 (xerophthalmia, and chalazion); and Notch signaling in meibomian gland epithelial cell differentiation, Investigative ophthalmology & visual science, 57(3):859-865 (meibomian gland dysfunction, and xerophthalmia).

By comparing the killing effect of a plurality of traditional Chinese medicinal materials on mites, it was found that Herba Taraxaci can effectively shorten survival time of mites. A variety of monomer compounds of Herba Taraxaci were further screened in experiments of killing mites. It was found that these monomer compounds can shorten the survival time of the mites compared with a control group, and in particular, taraxerol, diosmetin and taraxasterol acetate can significantly shorten the survival time of the mites, and can be used for preparing a product (such as a medicament, a cosmetic product, a daily product and the like) for killing or controlling mites. The use disclosed herein can effectively utilize natural resources and improve the utilization value and commercial value of Herba Taraxaci.

### DETAILED DESCRIPTION

Unless otherwise defined, all scientific and technical terms used herein have the same meaning as commonly understood by those skilled in the art to which the present invention relates.

Unless otherwise indicated, in the present invention, the Herba Taraxaci refers to Mongolian dandelion herb, a traditional Chinese medicinal material, which refers to a dried herb of *Taraxacum monolicium* Hand. -Mazz., *Taraxacum sinicum* Kitag. or several species of the same genus in the Asteraceae family. The processing steps for Herba Taraxaci comprises: removing impurities, washing, cutting into sections and drying in the sun. Herba Taraxaci is cold in nature and bitter and sweet in taste, is beneficial to liver and stomach meridians, and has effects of clearing heat and removing toxin, reducing swelling and removing stasis, promoting diuresis and relieving stranguria, etc.

The monomer of traditional Chinese medicine refers to a compound obtained from a single traditional Chinese medicine (e.g., by extraction), in particular, a compound with therapeutic effects or health benefits. For example, the monomer compound of Herba Taraxaci described herein refers to a monomer compound obtained from Herba Taraxaci, such as, but not limited to, taraxasterol (CAS: 1059-14-9), taraxerol (CAS: 127-22-0), taraxerone (CAS: 514-07-8), diosmetin (CAS: 520-34-3), taraxasterol acetate (CAS: 6426-43-3), caffeic acid (CAS: 331-39-5), taraxeryl acetate (CAS: 2189-80-2) and lupenone (CAS: 1617-70-5).

In the present invention, the term "animal" generally refers to vertebrates, in particular, mammals, including humans. The term "non-human animal" refers to any vertebrate except human beings, in particular, mammals. In some embodiments of the present invention, the non-human animal described herein is a domestic animal, i.e., an animal raised and domesticated by humans with artificially controlled reproduction for purposes such as food, labor, fur, companionship and experiment, such as an economic animal, a companion animal and a laboratory animal. The economic animal may be, for example, a livestock animal such as a pig, a cow, a sheep, a horse, a donkey, a fox, a raccoon dog, a mink, a camel and the like. The companion animal may be, for example, a dog, a cat, a rabbit, a murine (such as a guinea pig, a hamster, a gerbil, a chinchilla, a squirrel, etc.) and the like. The laboratory animal may be, for example, a monkey, a dog, a rabbit, a cat, a murine (such as a rat and a mouse) and the like.

The technical schemes of the present invention will be clearly and completely described below with reference to the examples of the present invention, and it is obvious that the described examples are only a part of the examples of the present invention but not all of them. Based on the examples of the present invention, all other examples obtained by those of ordinary skill in the art without creative work shall fall within the protection scope of the present invention.

### Example 1

### I. Methods:

### 1. Subjects

The ethical guidelines of "The Declaration of Helsinki" and "Measures for the Ethical Review of Biomedical Research Involving Humans" were strictly followed in this study. *Demodex* mites from patients diagnosed with ocular *Demodex* infestation were collected in this study. Subjects who met the inclusion criteria were enrolled after giving an informed conversation and signing an informed consent.

### 2. Detection of Demodex mites

The detection of *Demodex* mites was performed according to the conventional method of eyelash microscopy (12,20). 3 eyelashes were collected from each eyelid, with a total of 12 eyelashes. The collected eyelashes were immediately placed on glass slides with three eyelashes on each glass slide, and the glass slides were examined under a common optical microscope for observation. *Demodex* mites in all periods were counted and classified according to morphology (specific standard: *D. brevis* with a head-to-body ratio of 1:1, and *D. folliculorum* with a head-to-body ratio of 1:3 to 1:4). Only the adult mites completely exposed to the field of view were taken as experimental subjects (larvae and eggs were excluded from the study due to their fragility at early stage of life).

### 3. In-vitro culture of Demodex mites

On the basis of the above detection of *Demodex* mites, 35 µL of the solutions was separately added to the slides, and the survival of the mites was observed every 2 hours. During the experiment, the *Demodex* mites were observed by two skilled operators separately for the activities (bodies, limbs and the like) through the microscope to determine whether they were dead or not. If the two operators gave different results, the survival status was determined independently by a third skilled operator. The *in-vitro* culture was conducted in a climatic chamber at a temperature of 20 °C and a humidity of 96%. During the observation, the glass slides were transported in a wet box to ensure the high humidity.

### 4. Medicine

### 4.1 7 traditional Chinese medicines

The traditional Chinese medicines prepared in ready-to-use forms (Flos Lonicerae, Herba Taraxaci, Cortex Dictamni, Radix Scutellariae, Herba Houttuyniae, Cortex Fraxini and Flos Chrysanthemi) selected in the experiment were purchased from the traditional Chinese medicine pharmacy of Guangdong Provincial Hospital of Chinese Medicine. The Flos Lonicerae, Herba Taraxaci, Radix Scutellariae, Herba Houttuyniae and Flos Chrysanthemi were from Kangmei Pharmaceutical Co., Ltd., and the Cortex Dictamni and Cortex Fraxini were from Lingnan Traditional Chinese Medicine Tablets Co., Ltd.

### 4.2 Method for preparing traditional Chinese medicine decoction

60 g of dried ready-to-use forms was weighed using an electronic balance, and soaked in water with an amount of 10 times (600 mL) in a marmite overnight. The mixture was heated to boiling with strong fire, then decocted with small fire for 15-30 minutes (15 minutes for flowers and plants, and 30 minutes for rhizomes), and filtered with gauze. 500 mL of water was added to the filter residue, and the mixture was decocted in the same way and filtered. The two filtrates were combined in a beaker, and mixed well. The resulting mixture was heated and concentrated to a final volume of 100 mL, then cooled to room temperature, and transferred to a 100-mL volumetric flask and diluted to volume.

The treatment groups received different traditional Chinese medicine decoctions prepared by the above method, and the negative control group received sterilized water.

### 4.3 Positive control: tea tree oil and the major component terpinen-4-ol thereof

Tea tree oil (TTO) and terpinen-4-ol (T4O) were purchased from Milwaukee (WI, USA), and diluted in sterilized water to a final concentration of 10%.

### 5. Statistics

In this experiment, statistical analysis was conducted using SPSS22.0 statistical software, normality test was conducted using Shapiro-Wilk test, and homogeneity of variance test was conducted using Bartlett's test. The data with normal distribution and homogeneous variance were subjected to One-way ANOVA, and when there was statistical significance, Bonferroni's method was used for pairwise comparison among the groups; when the data did not converge to normal distribution, the Kruskal-Wallis test was adopted for statistical analysis with a test criterion of 0.05.

### 6. Results:

### 1. The Herba Taraxaci can significantly shorten the in-vitro survival time of Demodex mites

The *in-vitro* culture of the *Demodex* mites was conducted in an environment at a humidity of 96% and a temperature of 20 °C. The survival of the *Demodex* mites was observed dynamically and the survival time was recorded. The results are shown in Table 1. Compared with the negative control group, the *in-vitro* survival time of the *Demodex* mites in the groups of Herba Taraxaci was significantly shortened (21.61 ± 11.95 vs. 50.81 ± 19.90, P < 0.01), and was significantly shorter than that in the groups of TTO and T4O, which were currently considered to be effective in killing mites (21.61 ± 11.95 vs. 41.39 ± 19.33, 21.61 ± 11.95 vs. 40.50 ± 13.12, P < 0.05). The other 6 traditional Chinese medicines did not show significant differences in the *in-vitro* survival time of *Demodex* mites as compared with the negative control and the groups of TTO and T4O (P > 0.05). Interestingly, in this *in-vitro* culture experiment, TTO and its major active ingredient T4O, which were previously considered to be able to kill mites *in vitro,* did not show significant differences in the *in-vitro* survival time of *Demodex* mites compared with the negative control sterilized water (P > 0.05).

**Table 1: Effects of traditional Chinese medicine decoctions on survival time of D. folliculorum in vitro**

| Name of medicine | Survival time (hours) | Number of *Demodex* mites (N) |
|---|---|---|
| Water | 50.81 ±19.90 | 12 |
| TTO | 41.39 ± 19.33 | 22 |
| T4O | 40.50 ± 13.12 | 21 |
| Flos Lonicerae | 37.06 ± 23.43 | 14 |
| Herba Taraxaci | 21.61 ± 11.95^{aBC} | 13 |
| Flos Chrysanthemi | 31.33 ± 13.26 | 15 |
| Herba Houttuyniae | 49.57 ± 16.40 | 11 |
| Cortex Dictamni | 32.57 ± 19.80 | 18 |
| Radix Scutellariae | 34.13 ± 21.90 | 16 |
| Cortex Fraxini | 31.84 ± 15.12 | 22 |

Multiple comparisons were performed by Bonferroni's test. When compared with water, P^{A} denotes P < 0.05, P^{a} denotes P < 0.001, and P^{a} denotes P < 0.001; when compared with TTO, P^{B} denotes P < 0.05, P^{b} denotes P < 0.01, and P^{b} denotes P < 0.001; when compared with T4O, P^{C} denotes P < 0.05, P^{c} denotes p < 0.01, and P^{c'} denotes P < 0.001.

### Example 2: Preliminary screening in vitro of monomer compounds for effect on survival time of Demodex mites

### Methods:

### 1. Monomer compounds

According to the results of Example 1, Herba Taraxaci can significantly shorten the *in-vitro* survival time of *Demodex* mites, and thus monomer compounds of this traditional Chinese medicine were selected for further experiments. The names and molecular formulas of the monomer compounds are shown in the following table:
The monomer compounds were purchased from Chengdu Herbpurify Co., Ltd., as standard references.

**Table 2: Names and molecular formulas of monomer compounds**

| No. | Chinese name | Traditional Chinese medicine | English name | Molecular formula |
|---|---|---|---|---|
| 1 | 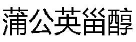 | Herba Taraxaci | Taraxasterol | C30H50O |
| 2 | 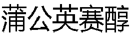 | Herba Taraxaci | Taraxerol | C30H50O |
| 3 | 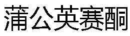 | Herba Taraxaci | Taraxerone | C30H48O |
| 4 | 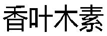 | Herba Taraxaci | Diosmetin | C16H12O6 |
| 5 | 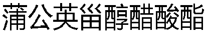 | Herba Taraxaci | Taraxasterol acetate | C32H52O2 |
| 6 | 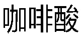 | Herba Taraxaci | Caffeic acid | C9H8O4 |
| 7 | 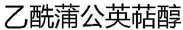 | Herba Taraxaci | Taraxeryl acetate | C32H52O2 |
| 8 | 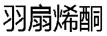 | Herba Taraxaci | Lupenone | C30H48O |

### 2. Preparation of monomer compound solutions

The powders of the monomer compounds shown in Table 2 were separately dissolved in dimethyl sulfoxide (DMSO), and then diluted with sterilized double-distilled water to make the final concentration of DMSO 10%, which could not shorten the survival time of Demodex mites as per a controlled study. The negative control group received DMSO mixed with sterilized water at a final concentration of 10%.

### 3. In-vitro culture of Demodex mites

As in Example 1, 35 µL of each solution was added to a glass slide, and the survival of the mites was observed under an optical microscope every 2 hours. During the experiment, the *Demodex* mites were observed by two skilled operators separately for the activities (bodies, limbs and the like) through the microscope to determine whether they were dead or not. If the two operators gave different results, the survival status was determined independently by a third skilled operator. The *in-vitro* culture was conducted in a climatic chamber at a temperature of 20 °C and a humidity of 96%. During the observation, the glass slides were transported in a wet box to ensure the high humidity.

### 4. Statistics

In this experiment, statistical analysis was conducted using SPSS22.0 statistical software, normality test was conducted using Shapiro-Wilk test, and homogeneity of variance test was conducted using Bartlett's test. The data with normal distribution and homogeneous variance were subjected to One-way ANOVA, and when there was statistical significance, Bonferroni's method was used for pairwise comparison among the groups; when the data did not converge to normal distribution, the Kruskal-Wallis test was adopted for statistical analysis with a test criterion α of 0.05.

### 5. Results:

Effects of monomer compounds of traditional Chinese medicine on survival time of *D. folliculorum* The *in-vitro* culture of the *Demodex* mites was conducted in an environment at a humidity of 96% and a temperature of 20 °C. The survival of the *Demodex* mites was observed dynamically and the survival time was recorded. The results are shown in Table 3. Compared with the negative control group receiving 10% DMSO solution, the monomer compounds of Herba Taraxaci could shorten the *in-vitro* survival time of *Demodex* mites, and in particular, the *in-vitro* survival time of *Demodex* mites was significantly shorter in the taraxerol group (37.91 ± 20.96 vs. 80.79 ± 25.34, P = 0.006), the diosmetin group (35.92 ± 14.82 vs. 80.79 ± 25.34, P = 0.001), and the taraxasterol acetate group (37.91 ± 20.96 vs. 80.79 ± 25.34, P < 0.001).

**Table 3: Effects of monomer compounds in traditional Chinese medicine on survival time of D. folliculorum in vitro**

| No. | Monomer compound | Survival time (hours) | Number of *Demodex* mites (N) |
|---|---|---|---|
| Control | 10%DMSO | 80.79 ±25.34 | 36 |
| 1 | Taraxasterol | 68.28±31.30 | 6 |
| 2 | Taraxerol | 37.91±20.96** | 11 |
| 3 | Taraxerone | 62.06±28.97 | 8 |
| 4 | Diosmetin | 35.92±14.82** | 10 |
| 5 | Taraxasterol acetate | 34.14±22.96*** | 13 |
| 6 | Caffeic acid | 66.45±24.24 | 5 |
| 7 | Taraxeryl acetate | 70.57±27.45 | 12 |
| 8 | Lupenone | 66.25±31.22 | 5 |

Multiple comparisons were conducted using Duncan's test; as compared with the control group, P*:p < 0.05, P**: p < 0.01, P***: p < 0.001.

### Example 3: Clinical study on treatment of xerophthalmia caused by mite infestation

### (I) Inclusion and exclusion criteria

### 1. Inclusion criteria

(1) Conformance to the diagnostic criteria of xerophthalmia based on the medical history.
(2) Aged 18-70 years;
(3) Any gender;
(4) Willingness to cooperate with treatment.

### 2. Exclusion criteria

(1) Iridocyclitis confirmed by slit-lamp examination;
(2) Ocular hypertension and hypotension;
(3) Ulcerative wounds on eyelid skin and corneal epithelial infiltrating lesions on corneal surface;
(4) Systemic disease, such as Sjogren's syndrome in patients with impaired hepatic and renal function;
(5) Aged <18 years or >70 years;
(6) Mental state improper for evaluation;
(7) Women in pregnancy and lactation.

### 3. Criteria for withdrawal and dropout

(1) Inability or unwillingness to continue the treatment due to other diseases accompanied in the treatment process;
(2) Inability to cooperate or unwillingness to continue the treatment due to worsening symptoms in the treatment process;
(3) Violation to the trial scheme and use of other medicines of this study;
(4) Inability to determine efficacy due to incomplete final data.

### (II) Endpoints

Ocular surface discomfort scoring, general ophthalmic examinations (vision, intraocular pressure, slit-lamp examination), xerophthalmia measurements (conjunctival hyperemia scoring, BUT test, and Schirmer I test), ocular surface disease index (OSDI) scoring, xerophthalmia detector and ocular *Demodex* examinations were performed on patients before the treatment and weekly after treatment.

### (III) Statistics

### 1. Estimation of sample size

Considering that xerophthalmia is a common clinical ocular surface disease, with positive rate of *Demodex* up to 23.8%-90.0%. According to the sample size calculation formula of superiority test on the basis of quantitative data, 30 cases were enrolled in each of the treatment and control groups.

### 2. Statistics and data analysis

SPSS20.0 software and Excel were used for statistical analysis, normally distributed enumeration data were analyzed by paired sample *t*-test, measurement data were analyzed by *_{χ}*2 test, and non-normally distributed data were analyzed by non-parametric test. The results were expressed as mean ± standard deviation, or x + s), and the differences were considered statistically significant when P < 0.05.

The above description is only for the purpose of illustrating the preferred example of the present invention, and is not intended to limit the scope of the present invention. Any modifications, equivalents and the like made without departing from the spirit and principle of the present invention shall fall within the protection scope of the present invention.

The foregoing examples and methods described herein may vary based on the abilities, experience, and preferences of those skilled in the art.

The certain order in which the procedures are described in the present invention does not constitute any limitation to the order of the procedures.

## Claims

1. Use of Herba Taraxaci in preparing a product for killing mites.

2. Use of a Herba Taraxaci extract in preparing a product for killing mites.

3. The use according to claim 2, wherein the Herba Taraxaci extract comprises one or more of taraxasterol, taraxerol, taraxerone, diosmetin, taraxasterol acetate, caffeic acid, taraxeryl acetate and lupenone;
preferably, the Herba Taraxaci extract comprises one or more of taraxerol, diosmetin and taraxasterol acetate.

4. Use of a monomer compound of Herba Taraxaci, or a salt, an isomer, a solvate or a derivative thereof in preparing a product for killing mites.

5. The use according to claim 4, wherein the monomer compound is selected from one or more of taraxasterol, taraxerol, taraxerone, diosmetin, taraxasterol acetate, caffeic acid, taraxeryl acetate and lupenone;
preferably, the monomer compound is selected from one or more of taraxerol, diosmetin and taraxasterol acetate.

6. Use of taraxerol in preparing a product for killing mites.

7. Use of diosmetin in preparing a product for killing mites.

8. Use of taraxasterol acetate in preparing a product for killing mites.

9. The use according to any one of claims 1-8, wherein the mites are one or more of *Demodex spp., Dermatophagoides pteronyssinus* and *Sarcoptes scabiei.*

10. The use according to any one of claims 1-8, wherein the product for killing mites is a pharmaceutical composition, a cosmetic product or an acaricide.

11. The use according to claim 10, wherein the pharmaceutical composition is a pharmaceutical composition for preventing and/or treating a disease caused by mite infestation;
the disease is selected from: an ocular disease, a skin disease and an allergic disease;
preferably, the ocular disease is selected from: blepharitis marginalis, meibomian gland dysfunction, tarsitis, madarosis, abnormal eyelash alignment, glaucoma, cataract, ocular folliculitis, conjunctivitis, blepharoconjunctivitis, pterygium, keratitis, eyelid laxity and ectropion, basal cell carcinoma of eyelid, xerophthalmia and chalazion;
preferably, the skin disease is selected from: seborrhoeic dermatitis, acne, acne rosacea, pityriasis folliculorum, perioral dermatitis, demodicosis, gaile and basal cell carcinoma; and
preferably, the allergic disease is selected from: allergic asthma, allergic rhinitis and allergic dermatitis.

12. The use according to claim 11, wherein the pharmaceutical composition is a topical preparation, preferably an ophthalmic preparation or a skin topical preparation;
preferably, the ophthalmic preparation is selected from: an eye drop, an eye ointment, an ophthalmic gel, an ophthalmic emulsion, an ophthalmic suspension, an ophthalmic film, a collyrium and an intraocular injection;
preferably, the skin topical preparation is selected from: an aerosol, a powder, a lotion, a tincture, a liniment, a film coating agent, an ointment, a gel, a paste and an emulsion.

13. The use according to claim 11, wherein the pharmaceutical composition is a human pharmaceutical composition or a veterinary pharmaceutical composition.

14. The use according to claim 10, wherein the cosmetic product is in a form selected from: a facial cleanser, a soap, a skin softener, a toner, a skin care lotion, a jelly lotion, a facial cream, a sunscreen cream, an essence, a facial mask, a gel, a foundation, a scrub, a neck cream, a shampoo, a shower gel, a hair conditioner, a body lotion, an eye cream, a mascara, an eyeliner powder, a cream eyeliner, an eyeliner pencil, an eye shadow powder, an eye shadow cream, an eyebrow pencil and a brow powder.

15. The use according to claim 10, wherein the acaricide is in a form selected from: a spray, a lotion, a paster and a small packet.

16. A method for treating and/or preventing an ocular disease caused by mites, comprising administering to eyes of a subject in need thereof Herba Taraxaci, a Herba Taraxaci extract, a monomer compound of Herba Taraxaci, or a salt, an isomer, a solvate or a derivative thereof.

17. Use of Herba Taraxaci, a Herba Taraxaci extract, a monomer compound of Herba Taraxaci, or a salt, an isomer, a solvate or a derivative thereof in preparing a medicament for treating and/or preventing an ocular disease caused by mites.
